(19) Europäisches Patentamt
European Patent Office
Office européen des brevets

(11) **EP 4 670 623 A1**

(12) **EUROPEAN PATENT APPLICATION**
published in accordance with Art. 153(4) EPC

(43) Date of publication:
**31.12.2025 Bulletin 2026/01**

(21) Application number: **24760359.0**

(22) Date of filing: **20.02.2024**

(51) International Patent Classification (IPC):
***A61B 5/022*** (2006.01)     ***A61B 5/02*** (2006.01)

(52) Cooperative Patent Classification (CPC):
**A61B 5/02; A61B 5/022**

(86) International application number:
**PCT/JP2024/006018**

(87) International publication number:
**WO 2024/177061 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.02.2023 JP 2023027217**

(71) Applicants:
• **Laview Corporation
Nagoya-shi, Aichi 464-0858 (JP)**

• **Matsumoto, Shingo
Tokyo 144-0051 (JP)**

(72) Inventors:
• **MASUDA, Hiroshi
Nagoya-shi, Aichi 464-0858 (JP)**
• **MATSUMOTO, Shingo
Tokyo 144-0051 (JP)**

(74) Representative: **FRKelly
Waterways House
Grand Canal Quay
Dublin D02 PD39 (IE)**

(54) **ARTERIOVENOUS PRESSURE MEASUREMENT DEVICE**

(57)     An arteriovenous pressure measurement device (10) includes a pressure detection unit (34), a volume detection unit (42), and an arithmetic unit (441). The pressure detection unit detects the internal pressure of a tourniquet. The volume detection unit detects the volume of a fluid in the tourniquet. The arithmetic unit computes the blood pressure of a subject based on the internal pressure of the tourniquet detected by pressure detection unit and the volume of the fluid in the tourniquet detected by the volume detection unit when the fluid is discharged from the inside of the tourniquet.

FIG.1

EP 4 670 623 A1

## Description

Cross Reference to Related Applications

The present application is based on and claims the benefit of the priority to Japanese Patent Application No. 2023-027217, filed February 24, 2023, the entire content of which is hereby incorporated by reference into this application.

Technical Field

The present disclosure relates to an arteriovenous pressure measurement device.

Background Art

There are cases where arterial pressure and venous pressure are measured by puncturing an artery and a vein or by using catheters, for example, to grasp the excess or deficiency of the circulating blood volume, a decrease in the cardiac function, a symptom of shock, a symptom of dehydration, and the like. A measurement device described in Patent Literature 1 below measures the central venous pressure of a subject by inserting a catheter into a blood vessel of the subject.

Citation List

Patent Literature

Patent Literature 1: Japanese Patent Laid-Open No. 2015-173952

Summary of Invention

To collect information on arterial pressure and venous pressure at the same time, it is necessary to use an invasive measurement method, such as inserting catheters into blood vessels of a subject as in the measurement device described in Patent Literature 1. However, such measurement involves the use of a dedicated device, for example, and thus is difficult to be adopted in small-scale clinics. Further, since such measurement is invasive, it is concerned that blood vessels may become damaged, or serious complications, such as embolism or infections, may occur, which in turn would increase burdens on patients.

An object of the present disclosure is to provide an arteriovenous pressure measurement device that can measure blood pressure with a simpler and safer method.

An arteriovenous pressure measurement device according to an aspect of the present disclosure includes a fluid inflow portion, a fluid discharge portion, a pressure detection unit, a volume detection unit, and an arithmetic unit. The fluid inflow portion flows a fluid to the inside of a tourniquet worn by a subject, thereby increasing the internal pressure of the tourniquet. The fluid discharge portion discharges the fluid from the inside of the tourniquet, thereby decreasing the internal pressure of the tourniquet. The pressure detection unit detects the internal pressure of the tourniquet. The volume detection unit detects the volume of the fluid in the tourniquet. The arithmetic unit computes the blood pressure of the subject based on the internal pressure of the tourniquet detected by pressure detection unit and the volume of the fluid in the tourniquet detected by the volume detection unit when the fluid is discharged from the inside of the tourniquet.

According to such a configuration, it is possible to detect the blood pressure of a subject without inserting a catheter or the like into the subject, that is, non-invasively. Thus, the measurement of the blood pressure can be made with a simpler and safer method.

Brief Description of Drawings

FIG. 1 is a block diagram illustrating the schematic configuration of an arteriovenous pressure measurement device of a first embodiment.

FIG. 2 is a graph illustrating the transition of the internal air volume of a tourniquet with respect to the internal pressure thereof when air is discharged from the tourniquet.

FIG. 3 is a graph illustrating the transition of the internal air volume of the tourniquet with respect to the internal pressure thereof when air is discharged from the tourniquet.

FIG. 4 is a graph illustrating the transition of a primary differential value with respect to the internal pressure of the tourniquet when air is discharged from the tourniquet.

FIG. 5 is a flowchart illustrating the procedures of processes executed by a control device of the first embodiment.

FIG. 6 is a graph illustrating the results of comparison between the measured values of blood pressure measured with the arteriovenous pressure measurement device of the first embodiment and the measured values of blood pressure of a reference example.

FIG. 7 is a graph illustrating the relationship between the internal pressure of the tourniquet and a secondary differential value when air is discharged from the tourniquet.

FIG. 8 is a flowchart illustrating the procedures of processes executed by a control device of a second embodiment.

FIG. 9 is a graph illustrating the transition of the internal air volume of the tourniquet with respect to the internal pressure thereof when air is discharged from the tourniquet.

FIG. 10 is a graph illustrating the results of comparison between the transitions of the internal pressure of the tourniquet and the secondary differential value with respect to the elapsed time when air is discharged from the tourniquet.

FIG. 11 is a graph illustrating the results of comparison between the transitions of the primary differential value and the secondary differential value with respect to the elapsed time when air is discharged from the tourniquet.

Description of Embodiments

Hereinafter, an embodiment of an arteriovenous pressure measurement device will be described with reference to the drawings. To help understand the description, components that are identical throughout the drawings are assigned identical reference signs as far as possible, and the description of such components shall be made only once.

<First embodiment>

(Configuration of arteriovenous pressure measurement device)

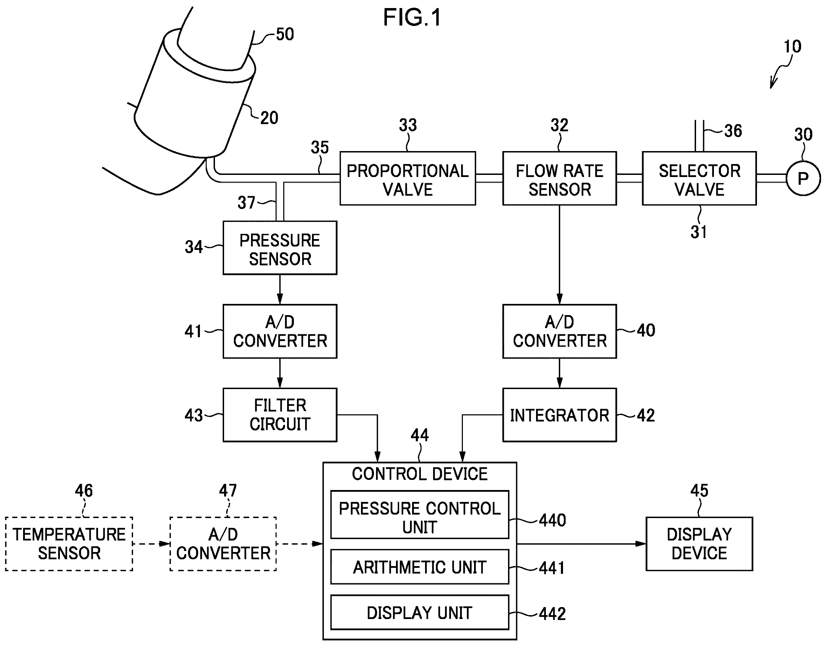

First, a first embodiment of an arteriovenous pressure measurement device will be described. An arteriovenous pressure measurement device 10 illustrated in FIG. 1 is a device that can non-invasively measure the pressure of a peripheral vein of a subject. As illustrated in FIG. 1, the arteriovenous pressure measurement device 10 includes a tourniquet 20, an air pump 30, a selector valve 31, a flow rate sensor 32, a proportional valve 33, and a pressure sensor 34.

The tourniquet 20 is a balloon-like member adapted to be wrapped around a part of a human body as a living organism, specifically, an upper arm 50. The tourniquet 20 is also referred to as a "cuff." The inside of the tourniquet 20 can be supplied with air. When the inside of the tourniquet 20 has been supplied with air, and the internal pressure of the tourniquet has thus increased, the tourniquet 20 compresses the upper arm 50. When the air is discharged from the inside of the tourniquet 20, the compression of the upper arm 50 by the tourniquet 20 is released.

The air pump 30 is connected to the tourniquet 20 through a pipe 35. The air pump 30 pumps air to the tourniquet 20 through the pipe 35. As the air pumped to the tourniquet 20 by the air pump 30 is supplied into the inside of the tourniquet 20, the internal pressure of the tourniquet 20 increases. As such, in the present embodiment, the air corresponds to a fluid supplied to the inside of the tourniquet 20. In addition, the air pump 30 corresponds to a fluid inflow portion that flows air to the inside of the tourniquet 20 to increase the internal pressure of the tourniquet 20.

The pipe 35 is provided with the selector valve 31, the flow rate sensor 32, and the proportional valve 33 in this order from the side of the air pump 30.

The selector valve 31 selectively switches between a supply state in which the pipe 35 is connected to the air pump 30 and a discharge state in which the pipe 35 is connected to a discharge pipe 36. When the selector valve 31 is in the supply state, air is supplied to the tourniquet 20 from the air pump 30 through the pipe 35. When the selector valve 31 is in the discharge state, air in the tourniquet 20 is discharged to the outside through the pipe 35 and the discharge pipe 36. As such, in the present embodiment, the selector valve 31 corresponds to a fluid discharge portion that discharges air from the inside of the tourniquet 20 to decrease the internal pressure of the tourniquet 20.

The flow rate sensor 32 detects the flow rate of air flowing through the pipe 35, and also outputs a signal corresponding to the detected flow rate of the air. When air is supplied to the tourniquet 20 from the air pump 30 through the pipe 35, the flow rate sensor 32 detects the air supply amount that is the flow rate of air supplied to the inside of the tourniquet 20. When air is discharged from the tourniquet 20 through the pipe 35 and the discharge pipe 36, the flow rate sensor 32 detects the air discharge amount that is the flow rate of air discharged from the inside of the tourniquet 20. In the present embodiment, the flow rate sensor 32 corresponds to a flow rate detection unit.

When air is supplied to the inside of the tourniquet 20 from the air pump 30 through the pipe 35, the proportional valve 33 controls the flow rate of the air supplied to the tourniquet 20. When air is discharged from the inside of the tourniquet 20 through the pipe 35 and the discharge pipe 36, the proportional valve 33 controls the flow rate of the air discharged from the tourniquet 20.

The pressure sensor 34 is provided on a branch pipe 37 branching from the pipe 35 at a position between the proportional valve 33 and the tourniquet 20. The pressure sensor 34 detects the pressure of air in the pipe 35 at the position between the proportional valve 33 and the tourniquet 20, that is, the internal pressure of the tourniquet 20. As such, in the present embodiment, the pressure sensor 34 corresponds to a pressure detection unit that detects the internal pressure of the tourniquet 20.

As illustrated in FIG. 1, the arteriovenous pressure measurement device 10 further includes A/D converters 40 and 41, an integrator 42, a filter circuit 43, a control device 44, and a display device 45.

The A/D converter 40 samples an output signal of the flow rate sensor 32. The A/D converter 40 converts the output signal of the flow rate sensor 32 at predetermined intervals, from an analog signal into a digital value. The digital value obtained through conversion indicates the value of the flow rate of air detected by the flow rate sensor 32, that is, the flow rate of air flowing through the pipe 35. The A/D converter 40 outputs the digital value of the flow rate of the air obtained through conversion to the integrator 42. Hereinafter, the digital value of the air flow rate Q obtained through conversion by the A/D converter 40 shall also be referred to as a "detected value of the air flow rate Q of the pipe 35."

The A/D converter 41 samples an output signal of the pressure sensor 34. The A/D converter 41 converts the output signal of the pressure sensor 34 at predetermined intervals, from an analog signal into a digital value. The digital value obtained through conversion indicates the value of the pressure of air detected by the pressure sensor 34, that is, the internal pressure of the tourniquet 20. The A/D converter 40 outputs the digital value of the internal pressure of the tourniquet 20 obtained through conversion to the filter circuit 43. Hereinafter, the digital value of the internal pressure of the tourniquet 20 obtained through conversion by the A/D converter 40 shall also be referred to as a "detected value of the internal pressure P of the tourniquet 20."

The integrator 42 integrates the detected values of the air flow rate Q of the pipe 35 sequentially output from the A/D converter 40, thereby computing the air volume VA that is the volume of air in the tourniquet 20. For example, the integrator 42 integrates the detected values of the air flow rate Q of the pipe 35 sequentially output from the A/D converter 40 from a time point when the supply of air from the air pump 30 to the tourniquet 20 is started to a time point when the supply of air is stopped, thereby computing the air volume VA of the tourniquet 20. As the time point when the supply of air to the tourniquet 20 is started, a time point when the state of the selector valve 31 is switched to the supply state to operate the air pump 30 is used, for example. As the time point when the supply of air to the tourniquet 20 is stopped, a time point when the air pump 30 is stopped is used, for example. The integrator 42 holds the air volume VA of the tourniquet 20 at the time point when the supply of air to the tourniquet 20 is stopped, as the maximum air volume Vmax.

Meanwhile, the integrator 42 integrates the detected values of the air flow rate Q of the pipe 35 sequentially output from the A/D converter 40 from a time point when the discharge of air from the tourniquet 20 is started, and also subtracts the integrated value from the maximum air volume Vmax, thereby computing the estimated value of the current air volume VA of the tourniquet 20. As the time point when the discharge of air from the tourniquet 20 is started, a time point when the state of the selector valve 31 is switched to the discharge state is used, for example.

As described above, the integrator 42 of the present embodiment computes the internal air volume VA of the tourniquet 20 not only when air is supplied to the tourniquet 20 but also when air is discharged from the tourniquet 20. The integrator 42 outputs to the control device 44 information on the computed current air volume VA of the tourniquet 20. As such, in the present embodiment, the integrator 42 corresponds to a volume detection unit that detects the volume of air in the tourniquet 20.

The filter circuit 43 smooths the detected values of the internal pressure P of the tourniquet 20 sequentially output from the A/D converter 41, and also outputs the smoothed detected values of the internal pressure P to the control device 44.

The control device 44 mainly includes a microcomputer having a CPU and a storage device, for example. The control device 44 includes a pressure control unit 440, an arithmetic unit 441, and a display unit 442 as the functional configurations implemented through the execution of programs stored in the storage device.

The pressure control unit 440 controls the operations of the air pump 30, the selector valve 31, and the proportional valve 33, and the like to change the internal pressure of the tourniquet 20. For example, the pressure control unit 440 operates the air pump 30 after switching the state of the selector valve 31 to the supply state, thereby increasing the internal pressure of the tourniquet 20 up to a predetermined pressure. At this time, the pressure control unit 440 controls the proportional valve 33 to control the flow rate of air supplied to the tourniquet 20. For example, the pressure control unit 440 controls the proportional valve 33 to allow air to be supplied to the tourniquet 20 at a constant flow rate per unit time. After that, the pressure control unit 440 switches the state of the selector valve 31 to the discharge state after stopping the air pump 30, thereby decreasing the internal pressure of the tourniquet 20. At this time, the pressure control unit 440 controls the proportional valve 33 to control the flow rate of air discharged from the tourniquet 20. For example, the pressure control unit 440 controls the proportional valve 33 to allow air to be discharged from the tourniquet 20 at a constant flow rate per unit time.

The arithmetic unit 441 monitors the air volume VA of the tourniquet 20 calculated by the integrator 42 and the internal pressure of the tourniquet 20 calculated by the filter circuit 43 from a time point when the discharge of air from the tourniquet 20 is started, specifically, from a time point when the state of the selector valve 31 is switched from the supply state to the discharge state. The arithmetic unit 441 computes a primary differential value, which is a value obtained by performing first-order differentiation on the air volume VA of the tourniquet 20 with respect to the internal pressure of the tourniquet 20, and also determines if the state of a blood vessel of the upper arm 50 compressed by the tourniquet 20 has changed from a flattened state to an open state based on the primary differential value. Upon detecting a change in the state of the blood vessel of the upper arm 50 compressed by the tourniquet 20 from a flattened state to an open state, the arithmetic unit 441 determines the internal pressure of the tourniquet 20 calculated by the filter circuit 43 at that time point, as the venous pressure of the subject.

The display unit 442 displays on the display device 45 information on the venous pressure of the subject computed by the arithmetic unit 441, for example. The display device 45 is a liquid crystal display or an organic EL display, for example.

(Principle of computation of blood pressure)

Next, the principle of the computation of blood pressure with the arithmetic unit 441 will be described.

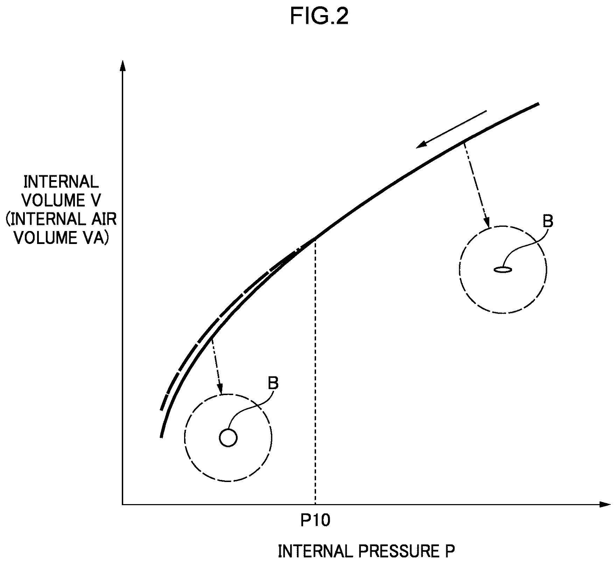

When the internal pressure of the tourniquet 20 has increased with air supplied to the tourniquet 20 to such an extent that the internal pressure of the tourniquet 20 becomes higher than the blood pressure of the subject, the blood vessel becomes a flattened state where it is compressed by the tourniquet 20. After that, when air is discharged from the tourniquet 20, the internal pressure P and the internal volume V of the tourniquet 20 change as illustrated in FIG. 2, for example.

As indicated by an arrow in FIG. 2, when air is discharged from the tourniquet 20, the internal pressure P and the internal volume V of the tourniquet 20 gradually decrease. Then, when the internal pressure P of the tourniquet 20 has become lower than the blood pressure, a blood vessel B transitions state from the flattened state to an open state as illustrated in FIG. 2. As the blood vessel B opens, the volume of the blood vessel B increases, which in turn causes a force of pushing the tourniquet 20 outward to act. Accordingly, the internal volume V of the tourniquet 20 decreases more significantly. For example, as illustrated in FIG. 2, provided that the blood vessel B has transitioned state from a flattened state to an open state when the internal pressure P of the tourniquet 20 has decreased down to "P10," the relationship between the internal pressure P and the internal volume V of the tourniquet 20 change as indicated by a solid line in FIG. 2. The change in the internal volume V of the tourniquet 20 with respect to the internal pressure P thereof indicated by the solid line is greater than a change in the internal volume V with respect to the internal pressure P indicated by a dashed and single-dotted line in FIG. 2. The dashed and single-dotted line corresponds to the extrapolation of the relationship between the internal pressure P and the internal volume V of the tourniquet 20 when the blood vessel B is in a flattened state, that is, when the internal pressure P of the tourniquet 20 is greater than "P10."

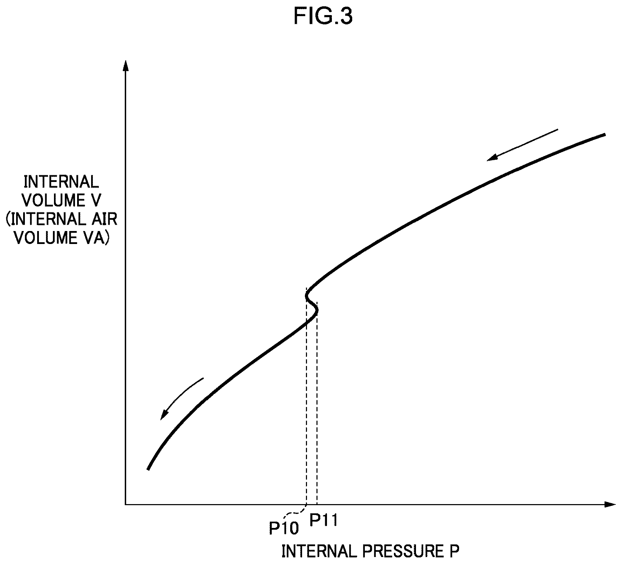

FIG. 3 illustrates an enlarged graph of portions of dashed and double-dotted lines in FIG. 2. When air is discharged from the tourniquet 20, the internal volume V of the tourniquet 20 as well as the internal pressure P of the tourniquet 20 decreases. Further, provided that a blood vessel starts to open when the internal pressure P of the tourniquet 20 has decreased down to "P10," the tourniquet 20 is pushed outward by the blood vessel, so that the internal volume V of the tourniquet 20 decreases more significantly, while the internal pressure P of the tourniquet 20 increases instantaneously. Therefore, as illustrated in FIG. 3, the internal pressure P of the tourniquet 20 instantaneously increases from "P10" to "P11," and then starts to decrease again. Thus, as long as changes in the internal pressure P of the tourniquet 20 are detectable, it is possible to accurately detect if a blood vessel has transitioned state from a flattened state to an open state. However, when the venous pressure of a subject is measured, there is a problem that the internal pressure P of the tourniquet 20 changes only slightly, and thus that such a change is difficult to detect.

Meanwhile, using variation in the internal volume V of the tourniquet 20 with respect to variation in the internal pressure P thereof, that is, using a primary differential value dV/dP of the internal volume V with respect to the internal pressure P can emphasize changes in the internal pressure P of the tourniquet 20, and thus can more reliably detect if a blood vessel has transitioned state from a flattened state to an open state. However, the internal volume V of the tourniquet 20 is difficult to detect directly. Thus, the arteriovenous pressure measurement device 10 of the present embodiment uses variation in the air volume VA of the tourniquet 20 instead of variation in the internal volume V of the tourniquet 20 to determine if a blood vessel has transitioned state from a flattened state to an open state. The reason that variation in the air volume VA of the tourniquet 20 can be used instead of variation in the internal volume V of the tourniquet 20 is as follows.

First, the internal pressure P and the internal volume V of the tourniquet 20, and the internal air mass m of the tourniquet 20 have the relationship of Expression f1 below. Note that in Expression f1, "R" represents the gas constant, and "T" represents the temperature in the tourniquet 20.

$$P \times V = m \times R \times T \quad (f1)$$

Performing differentiation on both sides of Expression f1 based on the internal pressure P can obtain Expression f2 below.

$$dV/dP = R \times T \times \{(dm/dP)/P - m/P^2\} \quad (f2)$$

Provided that the internal pressure P is sufficiently low, the primary differential value dV/dP can be approximated as in Expression f3 below.

$$dV/dP \approx (R \times T/P)(dm/dP) \quad (f3)$$

As is clear from Expression f3, variation in the internal volume V with respect to variation in the internal pressure P can be determined as variation in the internal air mass m of the tourniquet 20 with respect to variation in the internal pressure P. The internal air mass m of the tourniquet 20 is correlated with the air volume VA of the tourniquet 20. Consequently, variation in the internal volume V with respect to variation in the internal pressure P can be determined as variation in the air volume VA of the tourniquet 20 with respect to variation in the internal pressure P.

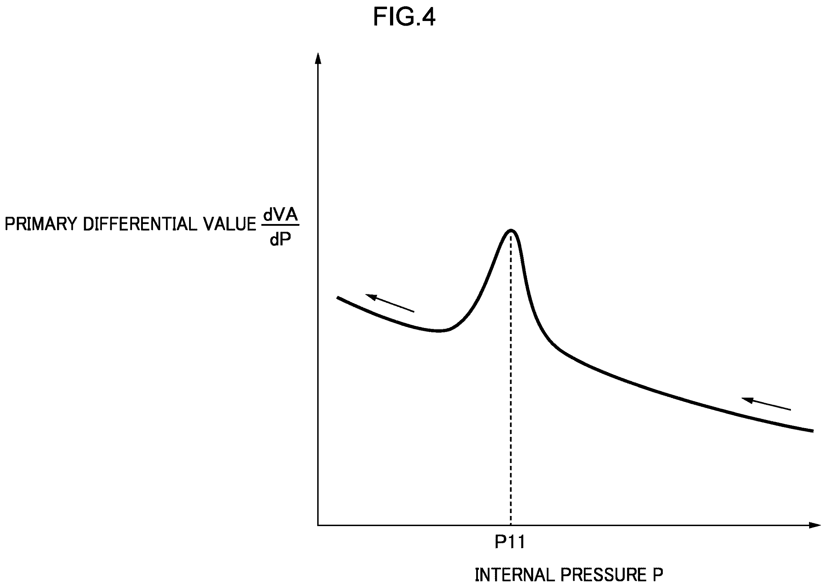

FIG. 4 is a graph illustrating the relationship between the primary differential value dVA/dP (on the ordinate axis) of the air volume VA of the tourniquet 20 with respect to the internal pressure P thereof, as determined when air is discharged from the tourniquet 20, and the internal pressure P (on the abscissa axis). As illustrated in FIG. 4, the primary differential value dVA/dP indicates the local maximum value when the internal pressure P indicates "P11," that is, when a blood vessel has transitioned state from a flattened state to an open state. Thus, using the internal pressure P when the primary differential value dVA/dP indicates the local maximum value can detect the blood pressure of a subject.

(Flow of processes executed by control device)

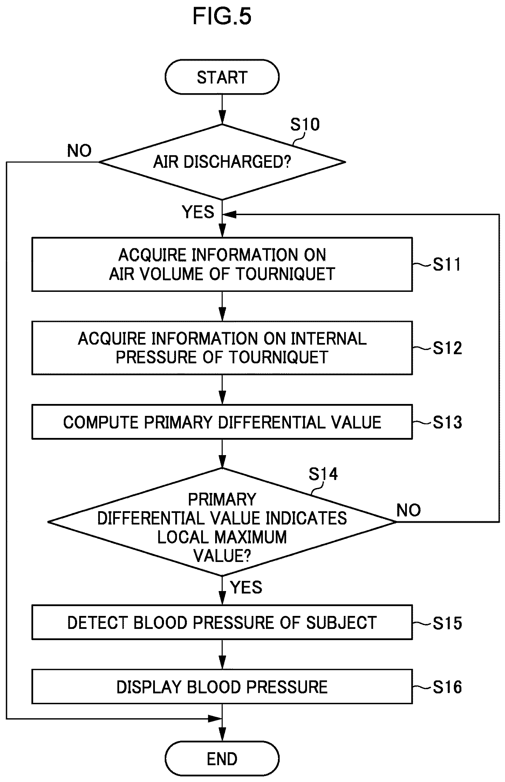

Next, specific procedures of a venous pressure measurement process executed by the control device 44 using the foregoing principle will be described with reference to FIG. 5. Note that the control device 44 repeatedly executes the processes illustrated in FIG. 5 with predetermined intervals.

As illustrated in FIG. 5, the arithmetic unit 441 of the control device 44 first determines if the discharge of air from the tourniquet 20 has been started (step S10). For example, if the selector valve 31 is in the supply state, the arithmetic unit 441 determines that the discharge of air from the tourniquet 20 has not been started (step S10: NO), and once terminates the processes illustrated in FIG. 5.

If the state of selector valve 31 is switched to the discharge state, the arithmetic unit 441 determines that the discharge of air from the tourniquet 20 has been started (step S10: YES). In such a case, the arithmetic unit 441 acquires from the integrator 42 information on the current air volume VA of the tourniquet 20 (step S11), and also acquires from the filter circuit 43 information on the current internal pressure P of the tourniquet 20 (step S12). Then, the arithmetic unit 441 computes the primary differential value dVA/dP (step S13), and determines if the computed primary differential value dVA/dP indicates the local maximum value (step S14). If the primary differential value dVA/dP does not indicate the local maximum value (step S14: NO), the arithmetic unit 441 returns back to the process of step S11, and thus acquires the air volume VA and the internal pressure P of the tourniquet 20, and also continues the computation of the primary differential value dVA/dP.

If the primary differential value dVA/dP indicates the local maximum value (step S14: YES), the arithmetic unit 441 detects the blood pressure of the subject based on the internal pressure P at that time (step S15). Then, the display unit 442 displays on the display device 45 the blood pressure of the subject detected by the arithmetic unit 441 (step S16).

(Operation and effect)

As described above, the arteriovenous pressure measurement device 10 of the present embodiment includes the air pump 30, the selector valve 31, the pressure sensor 34, the integrator 42, and the arithmetic unit 441. The air pump 30 flows air to the inside of the tourniquet 20 worn by a subject to increase the internal pressure of the tourniquet 20. The selector valve 31 discharges air from the inside of the tourniquet 20 to decrease the internal pressure of the tourniquet 20. The pressure sensor 34 detects the internal pressure of the tourniquet 20. The integrator 42 detects the volume VA of air in the tourniquet 20. The arithmetic unit 441 detects the blood pressure of the subject based on the internal pressure P of the tourniquet 20 detected by the pressure sensor 34 and the air volume VA of the tourniquet 20 detected by the integrator 42 when air is discharged from the inside of the tourniquet 20.

According to such a configuration, it is possible to detect the blood pressure of a subject without inserting a catheter or the like into the subject, that is, non-invasively. In addition, making the measurement of blood pressure with the foregoing method allows for the measurement of venous pressure that is a relatively low blood pressure in comparison with arterial pressure. Thus, the measurement of venous pressure can be made with a simpler method.

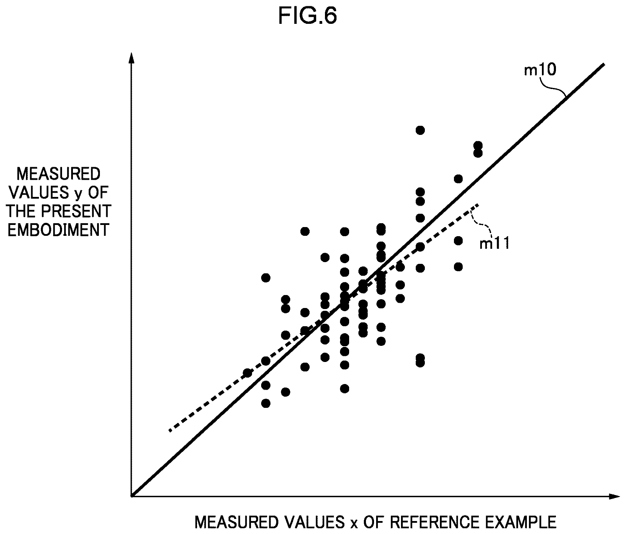

Further, the arteriovenous pressure measurement device 10 of the present embodiment can measure the blood pressure of a subject with about the same accuracy as the accuracy when the pressure of a venous blood vessel of the subject is directly measured with a catheter inserted into the subject using a pressure sensor. FIG. 6 illustrates the relationship between the measured values of the venous pressure of the subject detected by the arteriovenous pressure measurement device 10 of the present embodiment and the measured values obtained by directly measuring the venous pressure with a catheter. In FIG. 6, the measured values of the venous pressure of the subject detected by the arteriovenous pressure measurement device 10 of the present embodiment are described as the "measured values y of the present embodiment" on the ordinate axis, and the measured values obtained by directly measuring the venous pressure with a catheter are described as the "measured values x of a reference example" on the abscissa axis. In FIG. 6, the measured values y of the present embodiment with respect to the measured values x of the reference example when blood pressure measurement was conducted a plurality of times are indicated by dots.

In the graph illustrated in FIG. 6, a case where the measured values y of the present embodiment coincide with the measured values x of the reference example, that is, a function that satisfies "x=y" is indicated by a solid line m10. An approximate expression of the plurality of points illustrated in FIG. 6 was determined. Then, a straight line, such as the one indicated by a dashed line m11, was obtained. It is found that the dashed line m11 approximately coincides with the solid line m10. Thus, using the arteriovenous pressure measurement device 10 of the present embodiment can measure the blood pressure of a subject with about the same accuracy as the accuracy when venous pressure is directly measured with a catheter.

The arithmetic unit 441 computes the primary differential value dVA/dP that is a value obtained by performing first-order differentiation on the air volume VA of the tourniquet 20 with respect to the internal pressure P thereof. The arithmetic unit 441 computes the blood pressure of a subject based on the primary differential value dVA/dP and the internal pressure P of the tourniquet 20, more specifically, the internal pressure P when the primary differential value dVA/dP indicates the local maximum value.

According to such a configuration, it becomes easier to detect changes in the internal pressure P of the tourniquet 20. Thus, the blood pressure of a subject can be detected with higher accuracy.

The arteriovenous pressure measurement device 10 further includes the flow rate sensor 32. The flow rate sensor 32 detects the flow rate of air discharged from the inside of the tourniquet 20. The integrator 42 calculates the air volume VA of the tourniquet 20 based on the integrated value of the flow rates of air detected by the flow rate sensor 32 from a time point when the discharge of air from the inside of the tourniquet 20 is started.

According to such a configuration, the air volume VA of the tourniquet 20 can be calculated with high accuracy.

<Second embodiment>

Next, a second embodiment of the arteriovenous pressure measurement device 10 will be described. The following will mainly describe the differences from the arteriovenous pressure measurement device 10 of the first embodiment.

(Configuration of arteriovenous pressure measurement device)

The arteriovenous pressure measurement device 10 of the present embodiment detects if a blood vessel has transitioned state from a flattened state to an open state by using a secondary differential value $d^2VA/dP^2$, which is a value obtained by performing second-order differentiation on the air volume VA of the tourniquet 20 with respect to the internal pressure P thereof, instead of the primary differential value dVA/dP.

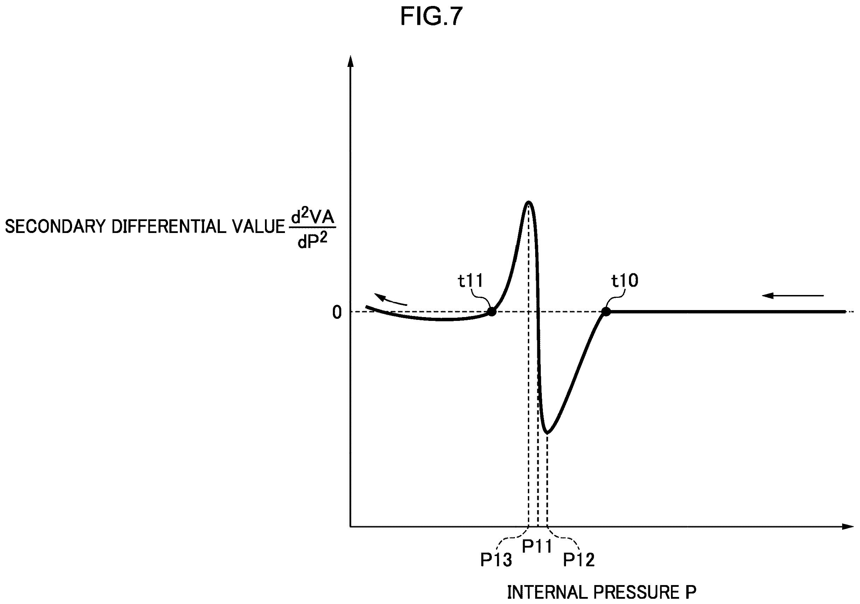

FIG. 7 is a graph illustrating the relationship between the secondary differential value $d^2VA/dP^2$ (on the ordinate axis) of the air volume VA of the tourniquet 20 with respect to the internal pressure P thereof, as determined when air is discharged from the tourniquet 20, and the internal pressure P (on the abscissa axis). As illustrated in FIG. 7, the secondary differential value $d^2VA/dP^2$ changes from a positive value to a negative value and then indicates a value of zero when the internal pressure P indicates "P11," that is, when a blood vessel has transitioned state from a flattened state to an open state. That is, the secondary differential value $d^2VA/dP^2$ crosses zero. Thus, using the internal pressure P when the secondary differential value $d^2VA/dP^2$ has crossed zero can detect the blood pressure of a subject.

(Flow of processes executed by control device)

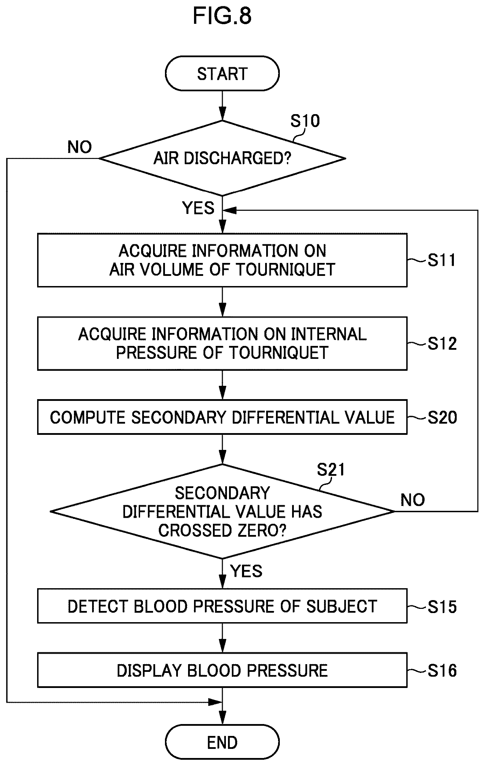

FIG. 8 illustrates specific procedures of a venous pressure measurement process executed by the control device 44 of the present embodiment. Note that among processes illustrated in FIG. 8, processes that are identical to the processes illustrated in FIG. 5 are assigned identical reference signs, and the description of such processes shall be made only once.

As illustrated in FIG. 8, the arithmetic unit 441 of the control device 44 computes the secondary differential value $d^2VA/dP^2$ (step S20) following the process of step S12, and then determines if the computed secondary differential value $d^2VA/dP^2$ has crossed zero (step S21). If the secondary differential value $d^2VA/dP^2$ has not crossed zero (step S21: NO), the arithmetic unit 441 returns back to the process of step S11. Meanwhile, if the secondary differential value $d^2VA/dP^2$ has crossed zero (step S21: YES), the arithmetic unit 441 executes a process of from step S15.

(Operation and effect)

As described above, in the arteriovenous pressure measurement device 10 of the present embodiment, the arithmetic unit 441 computes the secondary differential value $d^2VA/dP^2$ that is a value obtained by performing second-order differentiation on the air volume VA of the tourniquet 20 with respect to the internal pressure P thereof. The arithmetic unit 441 computes the blood pressure of a subject based on the secondary differential value $d^2VA/dP^2$ and the internal pressure P of the tourniquet 20, more specifically, the internal pressure P when the secondary differential value $d^2VA/dP^2$ has crossed zero.

According to such a configuration, it becomes easier to detect changes in the internal pressure P of the tourniquet 20. Thus, the blood pressure of a subject can be detected with higher accuracy.

(Modified example)

There may be a case where, depending on the state of a blood vessel of a subject and the like, a time point when the blood vessel transitions state from a flattened state to an open state deviates from a time point when the secondary differential value $d^2VA/dP^2$ has crossed zero. Therefore, it is also possible to detect the blood pressure of the subject based on, in the graph illustrated in FIG. 7, for example, an internal pressure P12 when the secondary differential value $d^2VA/dP^2$ indicates the local minimum value or an internal pressure P13 when the secondary differential value $d^2VA/dP^2$ indicates the local maximum value.

<Third embodiment>

Next, a third embodiment of the arteriovenous pressure measurement device 10 will be described. The following will mainly describe the differences from the arteriovenous pressure measurement device 10 of the first embodiment.

(Configuration of arteriovenous pressure measurement device)

The arteriovenous pressure measurement device 10 of the present embodiment further computes the volume of a lumen portion, such as a vein, compressed by the tourniquet 20.

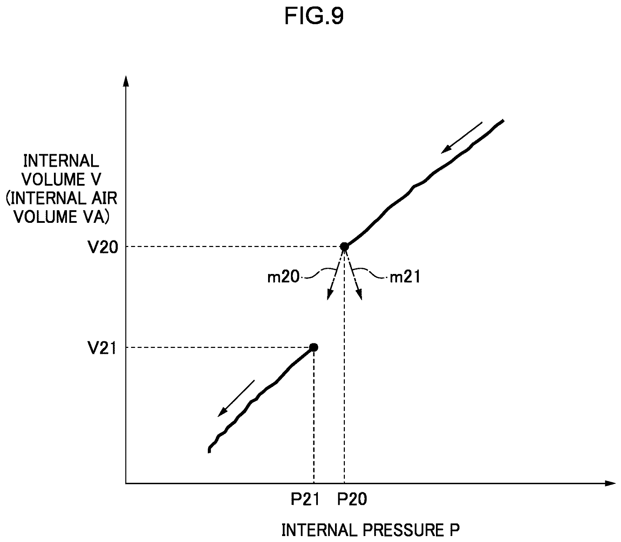

FIG. 9 is a graph illustrating the transition of the internal volume V of the tourniquet 20 with respect to the internal pressure P thereof when a lumen portion, such as a vein, opens as the internal pressure P of the tourniquet 20 decreases. In FIG. 8, the internal pressure P at a time point when the lumen portion in a flattened state has started to open is indicated by "P20," and the internal pressure P at a time point when the lumen portion has fully opened is indicated by "P21."

When the internal pressure P changes from P20 to P21, the relationship between the internal pressure P and the internal volume V of the tourniquet 20 changes as indicated by an arrow m20 or an arrow m21, for example, depending on the opening speed of the lumen portion. Specifically, if the opening speed of the lumen portion is low, the internal volume V of the tourniquet 20 changes with respect to the internal pressure P thereof as indicated by the arrow m20. Meanwhile, if the opening speed of the lumen portion is high, the internal volume V of the tourniquet 20 changes with respect to the internal pressure P thereof as indicated by the arrow m21.

If the internal volume V of the tourniquet 20 changes with respect to the internal pressure P thereof as indicated by the arrow m20, the secondary differential value $d^2VA/dP^2$ indicates a positive value when the internal pressure P starts to change from "P20." In contrast, if the internal volume V of the tourniquet 20 changes with respect to the internal pressure P thereof as indicated by the arrow m21, the secondary differential value $d^2VA/dP^2$ once indicates a negative value and then changes to a positive value when the internal pressure P starts to change from "P20." Thus, if the secondary differential value $d^2VA/dP^2$ indicates a positive value when the internal pressure P starts to change from "P20," the opening speed of the lumen portion can be determined to be low, and if the secondary differential value $d^2VA/dP^2$ indicates a negative value, the opening speed of the lumen portion can be determined to be high. In this manner, the opening speed of the lumen portion can be determined based on the sign of the secondary differential value $d^2VA/dP^2$ when the internal pressure P starts to change from "P20."

The absolute value of the primary differential value dVA/dP becomes greater as the difference between "P20" that is the internal pressure P at a time point when the lumen portion has started to open and "P21" that is the internal pressure P at a time point when the lumen portion has fully opened is smaller. Thus, it is possible to obtain information, such as the speed, that is, information on the opening process of the lumen portion from a time point when the lumen portion has started to open to a time point when the lumen portion has fully opened based on the absolute value of the primary differential value dVA/dP.

Further, it is also possible to compute the volume VL of the lumen portion compressed by the tourniquet 20 based on Expression f4 below, from "P20" that is the internal pressure P at a time point when the lumen portion has started to open and "P21" that is the internal pressure P at a time point when the lumen portion has fully opened. Note that in Expression f4, "V20" represents the internal volume of the tourniquet 20 at a time point when the internal pressure P20 is detected, and "V21" represents the internal volume of the tourniquet 20 at a time point when the internal pressure P21 is detected.

$$VL=V20-V21 \quad (f4)$$

Expression f4 above can be transformed into Expression f5 below by using f1 above. Note that in Expression f5, "m20" represents the internal air mass of the tourniquet 20 at a time point when the lumen portion has started to open, and "m21" represents the internal air mass of the tourniquet 20 at a time point when the lumen portion has fully opened.

$$VL=\{(m20/P20)-(m21/P21)\} \times R \times T \quad (f5)$$

As described above, the internal air mass m of the tourniquet 20 is correlated with the air volume VA of the tourniquet 20. Therefore, it is possible to use the air volume VA20 of the tourniquet 20 at a time point when the lumen portion has started to open instead of the internal air mass m20 of the tourniquet 20 at the time point when the lumen portion has started to open. In addition, it is also possible to use the air volume VA20 of the tourniquet 20 at a time point when the lumen portion has fully opened instead of the internal air mass m21 of the tourniquet 20 at the time point when the lumen portion has fully opened.

Meanwhile, the time point when the lumen portion has started to open, and the time point when the lumen portion has fully opened can be detected by using the secondary differential value $d^2VA/dP^2$. For example, the secondary differential value $d^2VA/dP^2$ changes as illustrated in FIG. 7. In such a case, at a time point t10 when the lumen portion starts to open, the secondary differential value $d^2VA/dP^2$ greatly changes in the negative direction from zero. Therefore, it is possible to determine that the lumen portion has started to open based on the fact that the secondary differential value $d^2VA/dP^2$ has greatly changed in the negative direction from zero. In addition, at a time point t11 when the secondary differential value $d^2VA/dP^2$ indicates zero after having changed from the negative value to a positive value, the lumen portion has fully opened. Therefore, it is possible to determine that the lumen portion has fully opened based on the fact that the secondary differential value $d^2VA/dP^2$ indicates zero after having crossed zero.

(Flow of processes executed by control device)

The control device 44 computes the volume VL of the lumen portion using the foregoing principle.

Specifically, as indicated by dashed lines in FIG. 1, the arteriovenous pressure measurement device 10 further includes a temperature sensor 46 and an A/D converter 47. The temperature sensor 46 detects the temperature of air flowing through the pipe 35, and also outputs a signal corresponding to the detected temperature of the air. The A/D converter 47 converts the output signal of the temperature sensor 46 from an analog signal into a digital value, and also transmits the digital value obtained through conversion to the control device 44.

The arithmetic unit 441 of the control device 44 acquires information on the temperature T of air flowing through the pipe 35 based on the digital value transmitted from the A/D converter 47.

The arithmetic unit 441 stores the air volume VA of the tourniquet 20 computed by the integrator 42 when the secondary differential value $d^2VA/dP^2$ has changed in the negative direction to a value greater than a predetermined value, as the air volume VA20 of the tourniquet 20 at a time point when the lumen portion has started to open. In addition, the arithmetic unit 441 stores the internal pressure P of the tourniquet 20 computed by the filter circuit 43 when the secondary differential value $d^2VA/dP^2$ has changed in the negative direction to a value greater than a predetermined value, as the internal pressure P20 of the tourniquet 20 at the time point when the lumen portion has started to open. In the present embodiment, the internal pressure P20 corresponds to a first internal pressure, and the air volume VA20 corresponds to a first fluid volume.

Further, the arithmetic unit 441 stores the air volume VA of the tourniquet 20 computed by the integrator 42 when the secondary differential value $d^2VA/dP^2$ indicates zero after having crossed zero, as the air volume VA21 of the tourniquet 20 at a time point when the lumen portion has fully opened. In addition, the arithmetic unit 441 stores the internal pressure P of the tourniquet 20 computed by the filter circuit 43 when the secondary differential value $d^2VA/dP^2$ indicates zero after having crossed zero, as the internal pressure P21 of the tourniquet 20 at the time point when the lumen portion has fully opened. In the present embodiment, the internal pressure P21 corresponds to a second internal pressure, and the air volume VA21 corresponds to a second fluid volume.

In addition, the arithmetic unit 441 computes the volume VL of the lumen portion based on the computed internal pressures P20 and P21, the computed air volumes VA20 and VA21, and the temperature T of air. The display unit 442 of the control device 44 displays on the display device 45 information on the volume VL of the lumen portion computed by the arithmetic unit 441.

(Operation and effect)

As described above, in the arteriovenous pressure measurement device 10 of the present embodiment, the arithmetic unit 441 detects an opening-start time point that is a time point when a lumen portion located at a part of a subject wearing the tourniquet 20 starts to open from a closed state, and a fully-open time point that is a time point when the lumen portion has fully opened based on the internal pressure P of the tourniquet 20 detected by the pressure sensor 34 and the air volume VA of the tourniquet 20 computed by the integrator 42 when pressure in the tourniquet 20 is decreased. In addition, the arithmetic unit 441 computes the volume VL of the lumen portion located at the part of the subject wearing the tourniquet 20 based on the first internal pressure P20 and the first air volume VA20 of the tourniquet 20 respectively detected by the pressure sensor 34 and the integrator 42 at the opening-start time point, and the second internal pressure P21 and the second air volume VA21 of the tourniquet 20 respectively detected by the pressure sensor 34 and the integrator 42 at the fully-open time point.

According to such a configuration, it is possible to further acquire information on the volume VL of a lumen portion of a subject. Further, as it is possible to grasp the volume VL of the lumen portion by watching the display device 45, it becomes further possible to recognize information on the blood volume of the subject.

<Fourth embodiment>

Next, a fourth embodiment of the arteriovenous pressure measurement device 10 will be described. The following will mainly describe the differences from the arteriovenous pressure measurement device 10 of the first embodiment.

(Configuration of arteriovenous pressure measurement device)

The arteriovenous pressure measurement device 10 of the present embodiment further computes information on compliance that represents the elasticity of a blood vessel.

Specifically, the compliance VC of a blood vessel can be computed with Expression f6 below. Note that in Expression f6, "$\Delta V$" represents variation in the internal volume V of the tourniquet 20 when the blood vessel transitions state from a flattened state to an open state, and "$\Delta P$" represents variation in the internal pressure P of the tourniquet when the blood vessel transitions state from a flattened state to an open state.

$$VC = \Delta V / \Delta P \quad (f6)$$

The variation $\Delta V$ in the internal volume of the tourniquet 20 and the variation $\Delta P$ in the internal pressure of the tourniquet 20 when the blood vessel transitions state from a flattened state to an open state can be respectively computed as indicated by Expressions f7 and f8 below, using the internal volume V20 and the internal pressure P20 of the tourniquet 20 at a time point when the lumen portion has started to open and the internal volume V21 and the internal pressure P21 of the tourniquet 20 at a time point when the lumen portion has fully opened that have been described in the third embodiment.

$$\Delta V = V20 - V21 \quad (f7)$$

$$|\Delta P = P20 - P21 \quad (f8)$$

As the variation $\Delta V$ in the internal volume of the tourniquet 20 represented by Expression f7, the volume VL of the lumen portion represented by Expression f4 above can be used. That is, the variation $\Delta V$ in the internal volume of the tourniquet 20 can be computed using f5 above.

(Flow of processes executed by control device)

The arithmetic unit 441 of the control device 44 computes the variation $\Delta V$ in the internal volume of the tourniquet 20 by using Expression f5 above. In addition, the arithmetic unit 441 computes the variation $\Delta P$ in the internal pressure of the tourniquet 20 by using Expression f8 above, from the internal pressure P20 of the tourniquet detected at a time point when a lumen portion has started to open and the internal pressure P21 of the tourniquet 20 at a time point when the lumen portion has fully opened. Further, the arithmetic unit 441 computes the compliance VC of the blood vessel based on Expression f6 above, from the variation $\Delta V$ in the internal volume and the variation $\Delta P$ in the internal pressure of the tourniquet 20 that have been computed. The display unit 442 of the control device 44 displays on the display device 45 information on the compliance VC of the blood vessel computed by the arithmetic unit 441.

(Operation and effect)

As described above, in the arteriovenous pressure measurement device 10 of the present embodiment, the arithmetic unit 441 computes the compliance VC of a blood vessel based on the volume VL of the lumen portion, the first internal pressure P20 of the tourniquet 20, and the second internal pressure P21 of the tourniquet 20.

According to such a configuration, it is possible to further acquire information on the compliance VC of a blood vessel of a subject. Further, as it is possible to grasp the compliance VC of the blood vessel of the subject by watching the display device 45, it becomes possible to more accurately learn the state of the blood vessel more accurately.

<Fifth embodiment>

Next, a fifth embodiment of the arteriovenous pressure measurement device 10 will be described. The following will mainly describe the differences from the arteriovenous pressure measurement device 10 of the first embodiment.

(Configuration of arteriovenous pressure measurement device)

The arteriovenous pressure measurement device 10 of the present embodiment further displays the breathing state of a subject on the display device 45.

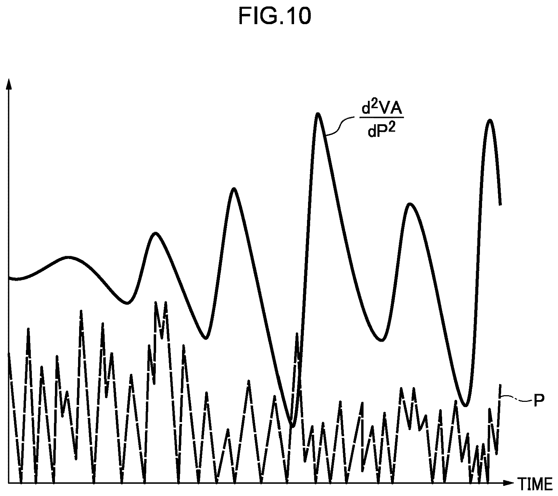

According to experiments conducted by the inventors, it has been confirmed that the foregoing secondary differential value $d^2VA/dP^2$ fluctuates due to not the influence of the throb of blood but the influence of the breathing of a subject. Specifically, it was possible to obtain a graph, such as the one illustrated in FIG. 10, as a result of experimentally measuring the temporal transition of the secondary differential value $d^2VA/dP^2$ and the transition of the internal pressure P of the tourniquet 20 along with the throb of an artery. In FIG. 10, the temporal transition of the secondary differential value $d^2VA/dP^2$ is indicated by a solid line, and the transition of the internal pressure P of the tourniquet 20 along with the throb of an artery is indicated by a dashed and single-dotted line. As is clear from FIG. 10, there is no correlation between the temporal change in the secondary differential value $d^2VA/dP^2$ and the temporal change in the internal pressure P of the tourniquet 20 along with the throb of an artery.

Meanwhile, from the periodicity of the temporal change in the secondary differential value $d^2VA/dP^2$, it is considered that the temporal change in the secondary differential value $d^2VA/dP^2$ is influenced by the breathing of a subject. This is considered to be because, for example, a vein connects to the lung through the pulmonary artery from the right atrium and the right ventricle, and thus, a change in pressure in the lung due to breathing causes a change in the pulmonary arterial pressure, which also has influence on the venous pressure. It has been also confirmed that the primary differential value $dVA/dP$ similarly fluctuates due to the influence of the breathing of a subject.

(Flow of processes executed by control device)

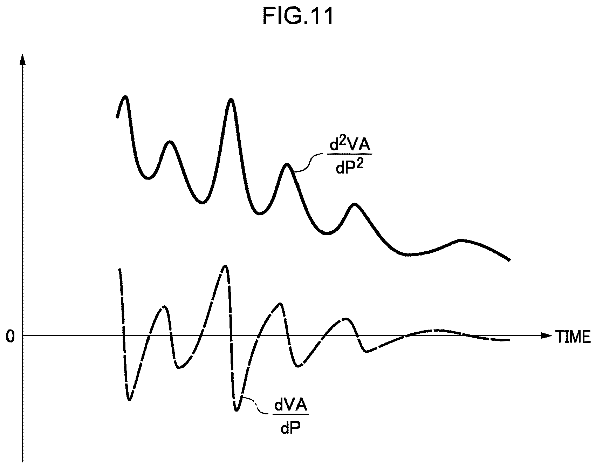

The display unit 442 of the present embodiment displays on the display device 45 the transition of the secondary differential value $d^2VA/dP^2$, such as the one indicated by a solid line in FIG. 11. In addition, when the blood pressure of a subject has been determined from the internal pressure P of the tourniquet 20 based on the secondary differential value $d^2VA/dP^2$, the display unit 442 displays each of the values of the internal pressure P of the tourniquet 20 and the secondary differential value $d^2VA/dP^2$ at the time of the determination of the blood pressure in a visible form.

Note that the display unit 442 may similarly display on the display device 45 the transition of the primary differential value $dVA/dP$, such as the one indicated by a dashed and single-dotted line in FIG. 11, and the values of the internal pressure P of the tourniquet 20 and the primary differential value $dVA/dP$ at the time of the determination of the blood pressure of the subject.

(Operation and effect)

As described above, in the arteriovenous pressure measurement device 10 of the present embodiment, the display unit 442 further displays the transition of the primary differential value $dVA/dP$, or the transition of the secondary differential value $d^2VA/dP^2$.

According to such a configuration, a medical expert, who is measuring the state of the blood pressure of a subject, for example, is able to confirm if the blood pressure has been appropriately detected in relation to the breathing state of the subject, by confirming the transition of the primary differential value $dVA/dP$ or the secondary differential value $d^2VA/dP^2$ displayed on the display device 45.

Note that the arteriovenous pressure measurement device 10 may be configured to be able to, if the medical expert determines that the timing of the detection of the blood pressure of the subject is not appropriate, correct the determined value of the internal pressure P of the tourniquet 20 to be used as the blood pressure of the subject. Accordingly, as the medical expert is able to freely change the determined value of the internal pressure P of the tourniquet 20 while confirming the actual transition of the primary differential value $dVA/dP$ or the secondary differential value $d^2VA/dP^2$, it is possible to measure the blood pressure of the subject with higher accuracy.

<Other embodiments>

Note that the foregoing embodiments can be implemented in the following form.

For example, the arteriovenous pressure measurement device 10 of each embodiment may be used not only to measure the venous pressure of a subject but also to measure the arterial pressure of the subject. Further, the arteriovenous pressure measurement device 10 may measure the venous pressure and the arterial pressure at the same time.

The present embodiment is not limited to the foregoing specific example. Any design change made as appropriate to the foregoing specific example by those skilled in the art is also included in the scope of the present disclosure as long as it includes the features of the present disclosure. The elements as well as their arrangement, conditions, and shapes, for example, of each of the foregoing specific examples are not limited to those exemplarily described above, and can be changed as appropriate. The combination of the elements of each of the foregoing specific examples can be changed as appropriate unless technical contradiction arises.

**Claims**

1. An arteriovenous pressure measurement device, comprising:

   a fluid inflow portion that flows a fluid to an inside of a tourniquet worn by a subject, thereby increasing an internal pressure of the tourniquet;
   a fluid discharge portion that discharges the fluid from the inside of the tourniquet, thereby decreasing the internal pressure of the tourniquet;
   a pressure detection unit that detects the internal pressure of the tourniquet;
   a volume detection unit that detects a volume of the fluid in the tourniquet; and
   an arithmetic unit that computes a blood pressure of the subject based on the internal pressure of the tourniquet detected by the pressure detection unit and the volume of the fluid in the tourniquet detected by the volume detection unit when the fluid is discharged from the inside of the tourniquet.

2. The arteriovenous pressure measurement device according to claim 1, wherein the arithmetic unit computes a venous pressure of the subject as the blood pressure of the subject.

3. The arteriovenous pressure measurement device according to claim 1, wherein the arithmetic unit computes an arterial pressure of the subject as the blood pressure of the subject.

4. The arteriovenous pressure measurement device according to claim 1, wherein the arithmetic unit

   computes a primary differential value that is a value obtained by performing first-order differentiation on the volume of the fluid in the tourniquet with respect to the internal pressure of the tourniquet, and
   computes the blood pressure of the subject based on the primary differential value and the internal pressure of the tourniquet.

5. The arteriovenous pressure measurement device according to claim 4, wherein the arithmetic unit computes the blood pressure of the subject based on the internal pressure of the tourniquet when the primary differential value indicates a local maximum value.

6. The arteriovenous pressure measurement device according to claim 4, further comprising a display unit that displays a transition of the primary differential value.

7. The arteriovenous pressure measurement device according to claim 1, wherein the arithmetic unit

   computes a secondary differential value that is a value obtained by performing second-order differentiation on the volume of the fluid in the tourniquet with respect to the internal pressure of the tourniquet, and
   computes the blood pressure of the subject based on the secondary differential value and the internal pressure of the tourniquet.

8. The arteriovenous pressure measurement device according to claim 7, wherein the arithmetic unit computes the blood pressure of the subject based on the internal pressure of the tourniquet when the secondary differential value has crossed zero.

9. The arteriovenous pressure measurement device according to claim 7, wherein the arithmetic unit computes the blood pressure of the subject based on the internal pressure of the tourniquet when the secondary differential value indicates a local minimum value.

10. The arteriovenous pressure measurement device according to claim 7, further comprising a display unit that displays a transition of the secondary differential value.

11. The arteriovenous pressure measurement device according to claim 1, further comprising a flow rate detection unit that detects a flow rate of the fluid discharged from the inside of the tourniquet, wherein the volume detection unit calculates the volume of the fluid in the tourniquet based on an integrated value of flow rates of the fluid detected by the flow rate detection unit.

12. The arteriovenous pressure measurement device according to claim 1, wherein the arithmetic unit

   detects, based on the internal pressure of the tourniquet detected by the pressure detection unit and the volume of the fluid in the tourniquet detected by the volume detection unit when the fluid is discharged from the inside of the tourniquet, an opening-start time point that is a time point when a lumen portion located at a part of the subject wearing the tourniquet starts to open from a closed state, and a fully-open time point that is a time point when the lumen portion has fully opened, and
   further computes a volume of the lumen portion located at the part of the subject wearing the tourniquet based on a first internal pressure and a first fluid volume of the tourniquet respectively detected by the pressure detection unit and the volume detection unit at the opening-start time point, and a second internal pressure and a second fluid volume of the tourniquet respectively detected by the pressure detection unit and the volume detection unit at the fully-open time point.

13. The arteriovenous pressure measurement device according to claim 12, wherein the arithmetic unit further computes a compliance of a blood vessel based on the volume of the lumen portion, the first internal pressure, and the second internal pressure.

# FIG.1

EP 4 670 623 A1

# FIG.2

INTERNAL VOLUME V (INTERNAL AIR VOLUME VA)

P10

INTERNAL PRESSURE P

FIG.3

INTERNAL
VOLUME V
(INTERNAL AIR
VOLUME VA)

P10   P11

INTERNAL PRESSURE P

## FIG.4

PRIMARY DIFFERENTIAL VALUE $\dfrac{dVA}{dP}$

P11

INTERNAL PRESSURE P

EP 4 670 623 A1

# FIG.5

```
                    ┌──────────────┐
                    │    START     │
                    └──────┬───────┘
                           │
                           ▼              S10
          NO      ◇───────────────────◇
    ┌──────────────  AIR DISCHARGED?
    │               ◇───────────────────◇
    │                        │ YES  ◄──────────────────────┐
    │               ┌─────────────────────────────────┐    │
    │               │  ACQUIRE INFORMATION ON         │    │
    │               │  AIR VOLUME OF TOURNIQUET        │~S11│
    │               └─────────────────┬───────────────┘    │
    │                                 ▼                     │
    │               ┌─────────────────────────────────┐    │
    │               │  ACQUIRE INFORMATION ON INTERNAL │    │
    │               │  PRESSURE OF TOURNIQUET          │~S12│
    │               └─────────────────┬───────────────┘    │
    │                                 ▼                     │
    │               ┌─────────────────────────────────┐    │
    │               │  COMPUTE PRIMARY DIFFERENTIAL VALUE │~S13│
    │               └─────────────────┬───────────────┘    │
    │                                 ▼          S14        │
    │                         ◇───────────────────◇        │
    │                      ◇   PRIMARY             ◇   NO   │
    │                    ◇  DIFFERENTIAL VALUE INDICATES ◇──┘
    │                      ◇  LOCAL MAXIMUM        ◇
    │                        ◇   VALUE?          ◇
    │                         ◇───────┬─────────◇
    │                                 │ YES
    │               ┌─────────────────────────────────┐
    │               │  DETECT BLOOD PRESSURE OF SUBJECT │~S15
    │               └─────────────────┬───────────────┘
    │                                 ▼
    │               ┌─────────────────────────────────┐
    │               │  DISPLAY BLOOD PRESSURE          │~S16
    │               └─────────────────┬───────────────┘
    │                                 │
    └─────────────────────────────────┤
                                      ▼
                             ┌──────────────┐
                             │     END      │
                             └──────────────┘
```

## FIG.6

MEASURED VALUES y OF THE PRESENT EMBODIMENT

m10

m11

MEASURED VALUES x OF REFERENCE EXAMPLE

# FIG.7

EP 4 670 623 A1

## FIG.8

```
                    ┌─────────┐
                    │  START  │
                    └─────────┘
                         │
                         ▼              S10
                    ╱─────────────╲
          NO       ╱   AIR          ╲
      ┌───────────╱  DISCHARGED?     ╲
      │           ╲                   ╱
      │            ╲─────────────────╱
      │                    │
      │                   YES ◄───────────────────┐
      │                    ▼                       │
      │         ┌──────────────────────────┐       │
      │         │   ACQUIRE INFORMATION ON  │      │
      │         │  AIR VOLUME OF TOURNIQUET │ ~S11 │
      │         └──────────────────────────┘       │
      │                    │                        │
      │                    ▼                        │
      │         ┌──────────────────────────┐        │
      │         │ ACQUIRE INFORMATION ON    │        │
      │         │ INTERNAL PRESSURE OF      │ ~S12   │
      │         │ TOURNIQUET                │        │
      │         └──────────────────────────┘        │
      │                    │                         │
      │                    ▼                         │
      │         ┌──────────────────────────┐         │
      │         │ COMPUTE SECONDARY         │ ~S20    │
      │         │ DIFFERENTIAL VALUE        │         │
      │         └──────────────────────────┘         │
      │                    │                          │
      │                    ▼           S21            │
      │              ╱─────────────╲                  │
      │             ╱  SECONDARY     ╲     NO          │
      │            ╱ DIFFERENTIAL     ╲────────────────┘
      │            ╲ VALUE HAS        ╱
      │             ╲ CROSSED ZERO?  ╱
      │              ╲──────────────╱
      │                    │
      │                   YES
      │                    ▼
      │         ┌──────────────────────────┐
      │         │  DETECT BLOOD PRESSURE   │ ~S15
      │         │  OF SUBJECT              │
      │         └──────────────────────────┘
      │                    │
      │                    ▼
      │         ┌──────────────────────────┐
      │         │  DISPLAY BLOOD PRESSURE  │ ~S16
      │         └──────────────────────────┘
      │                    │
      └────────────────────┤
                           ▼
                    ┌─────────┐
                    │   END   │
                    └─────────┘
```

FIG.9

FIG.10

FIG.11

## INTERNATIONAL SEARCH REPORT

| International application No. |
| --- |
| **PCT/JP2024/006018** |

| **A.** | **CLASSIFICATION OF SUBJECT MATTER** |
| --- | --- |

*A61B 5/022*(2006.01)i; *A61B 5/02*(2006.01)i
FI:  A61B5/022 100A; A61B5/02 A

According to International Patent Classification (IPC) or to both national classification and IPC

| **B.** | **FIELDS SEARCHED** |
| --- | --- |

Minimum documentation searched (classification system followed by classification symbols)

A61B5/02-5/03

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| **C.** | **DOCUMENTS CONSIDERED TO BE RELEVANT** | |
| --- | --- | --- |

| Category* | Citation of document, with indication, where appropriate, of the relevant passages | Relevant to claim No. |
| --- | --- | --- |
| X | JP 6651087 B1 (NATIONAL UNIVERSITY CORPORATION NAGOYA UNIVERSITY) 19 February 2020 (2020-02-19) paragraphs [0017]-[0037], [0043]-[0052], fig. 1-5 | 1-3, 11 |
| A | JP 6-319707 A (OMRON CORPORATION) 22 November 1994 (1994-11-22) paragraphs [0019]-[0034], fig. 1-10 | 1-13 |
| A | JP 2009-279196 A (OMRON HEALTHCARE CO., LTD.) 03 December 2009 (2009-12-03) paragraphs [0033]-[0064], fig. 1-21 | 1-13 |
| A | US 5570694 A (HEWLETT-PACKARD COMPANY) 05 November 1996 (1996-11-05) column 8, line 39 to column 13, line 2, fig. 1-8 | 1-13 |

☐ Further documents are listed in the continuation of Box C.      ☑ See patent family annex.

| | |
| --- | --- |
| \*    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "D"  document cited by the applicant in the international application | "X"  document of particular relevance; the claimed invention cannot be considered novel or cannot be considered to involve an inventive step when the document is taken alone |
| "E"  earlier application or patent but published on or after the international filing date | |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **25 April 2024** | **14 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/006018**

| Patent document cited in search report | | | Publication date (day/month/year) | Patent family member(s) | | | Publication date (day/month/year) |
|---|---|---|---|---|---|---|---|
| JP | 6651087 | B1 | 19 February 2020 | US paragraphs [0024]-[0044], [0050]-[0059], fig. 1-5 WO EP | 2022/0031179 2020/121377 3895608 | A1 A1 A1 | |
| JP | 6-319707 | A | 22 November 1994 | (Family: none) | | | |
| JP | 2009-279196 | A | 03 December 2009 | US paragraphs [0128]-[0203], fig. 1-44 WO CN | 2011/0125035 2009/142266 102036604 | A1 A1 A | |
| US | 5570694 | A | 05 November 1996 | DE column 6, line 12 to column 10, line 62, fig. 1-8 | 4331451 | C1 | |

Form PCT/ISA/210 (patent family annex) (July 2022)

**EP 4 670 623 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023027217 A
- JP 2015173952 A